# EUROPÄISCHE PATENTANMELDUNG

(11) **EP 1 961 408 A2**
(43) Veröffentlichungstag der Anmeldung: **27.08.2008**
(21) Anmeldenummer: 08100921.9
(22) Anmeldetag: 25.01.2008
(51) Int. Cl.: A61K 8/02, A61K 8/44, A61K 8/97, A61K 8/35, A61K 8/49, A61K 8/46, A61K 8/67, A61K 8/36, A61Q 19/00, A61Q 19/06

(54) **Kosmetisches Kombinationsprodukt zur Verbesserung des äußeren Erscheinungsbildes**

(30) Priorität: 26.02.2007 DE 102007009650
(71) Anmelder: Beiersdorf Aktiengesellschaft, D-20253 Hamburg (DE)
(72) Erfinder: Kröpke, Rainer, 22869, Schenefeld (DE); Fänger, Sabine, 22763, Hamburg (DE); Koop, Urte, 20149, Hamburg (DE); Eckert, Julia, 22089, Hamburg (DE); Schulz, Jens, 22869, Schenefeld (DE); Schepky, Andreas, 25474, Bönningstedt (DE)

(57) **Zusammenfassung**

Die vorliegende Erfindung betrifft ein kosmetisches Kombinationsprodukt umfassend ein oder mehrere oral einnehmbare Nahrungsergänzungsmittel in Kombination mit einer oder mehreren topisch applizierbaren kosmetischen Zubereitungen und/oder mit einer oder mehreren auf der Haut applizierbaren Hautauflagen. Die Kombinationsproduktbestandteile, die Nahrungsergänzungsmittel, die Zubereitungen und die Hautauflagen, zeichnen sich dadurch aus, dass sie individuell wählbar aufeinander abgestimmte Inhaltstoffe umfassen.

## Beschreibung

Die vorliegende Erfindung betrifft ein kosmetisches Kombinationsprodukt umfassend ein oder mehrere oral einnehmbare Nahrungsergänzungsmittel in Kombination mit einer oder mehreren topisch applizierbaren kosmetischen Zubereitungen und/oder mit einer oder mehreren auf der Haut applizierbaren Hautauflagen.
Die Kombinationsproduktbestandteile, die Nahrungsergänzungsmittel, die Zubereitungen und die Hautauflagen, zeichnen sich dadurch aus, dass sie individuell wählbar aufeinander abgestimmte Inhaltstoffe umfassen.
Es ist somit erstmals möglich kosmetische Hautpflege oder -verschönerung in zwei bzw. drei Anwendungsformen anzubieten und zu kombinieren - von aussen und/oder innen, kurzfristig oder permanent bzw. zeitlich begrenzt.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Die Haut ist sich ständig verändernden Umwelteinflüssen ausgesetzt und unterliegt auch im Laufe der Zeit einer Reihe von Veränderungen. So kommt es zu Veränderungen der Barriereeigenschaften, der Hautfaltigkeit- und Elastizität, der Pigmentierung und insbesondere infolge exogener Einflusse auch zu unterschiedlichen entzündlichen Reaktionen und beispielsweise auch zu Nachreaktionen der Haut auf die Einwirkung von UV-Strahlung.
Die Barrierewirkung der Haut kann über die Bestimmung des transepidermalen Wasserverlustes (TEWL - transepidermal water loss) quantifiziert werden. Dabei handelt es sich um die Abdunstung von Wasser aus dem Körperinneren ohne Einbeziehung des Wasserverlustes beim Schwitzen. Die Bestimmung des TEWL-Wertes hat sich als außerordentlich informativ erwiesen und kann zur Diagnose rissiger oder schrundiger Haut, zur Bestimmung der Verträglichkeit chemisch verschiedenartig aufgebauter Tenside und dergleichen mehr herangezogen werden.

Für die Schönheit und Gepflegtheit der Haut ist der Wasseranteil in der obersten Hautschicht von größter Bedeutung. Man kann ihn in einem begrenzten Umfang durch Einbringen von Feuchtigkeitsregulatoren günstig beeinflussen.

Unter kosmetischer Hautpflege ist in erster Linie zu verstehen, dass die natürliche Funktion der Haut als Barriere gegen Umwelteinflüsse, z.B. Schmutz, Chemikalien, Mikroorganismen, und gegen den Verlust von körpereigenen Stoffen, z.B. Wasser, natürliche Fette, Elektrolyte, gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Es sind seit Jahrhunderten kosmetische Cremes und Lotionen bekannt, die einen pflegenden Einfluss auf die Haut ausüben.

So sind beispielsweise aus DE 102005032237 kosmetische Zubereitungen bekannt, die insbesondere als Anti-Cellulite Mittel angewendet werden können.

Bei Cellulite (medizinischer Begriff: Dermopanniculosis) handelt es sich nicht um eine Krankheit, sondern um ein kosmetisches Problem.

Die Ursachen für Cellulite liegen vor allem am speziellen Aufbau der weiblichen Haut und an der Reaktion auf die weiblichen Hormone. In der Unterhaut sind Fettzellen gespeichert. Deren Menge wird schon im Säuglingsstadium festgelegt und ist durch Ernährung oder Sport nicht beeinflussbar. In den Fettzellen werden die Fettsäuren aus der Nahrung in Fette umgewandelt und knötchenförmig ins Bindegewebe eingelagert.

Wenn diese Fette über längere Zeit nicht abgebaut werden (z.B. Sport), und wird der Körper zusätzlich überernährt, können sich die Zellen um ein Vielfaches Ihrer Größe ausdehnen. Die vergrößerten Zellen drücken sich dann durch das Bindegewebe und es kommt zur gefürchteten Orangenhaut, auch als Cellulite bezeichnet.

Die weiteren Folgen im Alter sind Besenreiser, Krampfadern, Thrombosen und Beinleiden. Oft sind die Oberschenkel auch Speicher für überflüssiges Fett, dass über die Nahrung aufgenommen wird. Die unschönen, seitlichen Verdickungen der Oberschenkel, auch Reiterhosen genannt, kombiniert mit Cellulitis stellen oft eine große Belastung für die Betroffene dar.

Bekannt zur Behandlung der Cellulite ist beispielsweise das sog. body-wrap Prinzip.

Bekannt sind auch Anti-Cellulite-Pflaster (zum Beispiel "Nivea Good by Cellulite-Patch®" von Beiersdorf).
Aus WO 05/007127, WO 04/093865, WO 04/074216 und WO 04/060268 werden ebenfalls zahlreiche kosmetische Wirkstoffe und Behandlungsmethoden zur Behandlung von Cellulite aufgezeigt.
In EP 1181926, EP 728472 und EP 493151 werden zur Cellulitebehandlung insbesondere Coffein-haltige Kosmetika offenbart.
DE 102005053909 beschreibt selbstklebende Hautauflage, die mittels bestimmter Wirkstoffe in Kombinaton mit einer hautfreundlichen Klebemasse als Anti-Cellulite patches wirken.

Auch Nahrungsergänzungsmittel als sog. "Kosmetik von Innen" sind zur Hautpflege und - verschönerung bekannt.
So sind beispielsweise aus WO 2006023342, EP1711070, EP1218001, WO2006076240, EP1729851, EP0950410, EP 0731699, EP 0579901 oder EP1605764 Nahrungsergänzungsmittel bekannt, die u.a. auch gegen die Erscheinungen der Cellulite Anwendung finden.

Bei den drei bekannten Anwendungsformen, der Nahrungsergänzung, der kosmetischen Zubereitung und/oder der Hautauflage, steht der Anwender vor dem Problem sein individuelles Hautproblem gezielt bekämpfen zu können. Er ist dabei häufig überfordert aufgrund der verschiedensten Produkte, unterschiedlichen Wirkstoffen und differienden Anwendungshinweisen die geeigneten Produkte auszuwählen.

Jedes einzelne Produkt wirkt häufig immer nur auf eine Weise und die Wirkstoffe sind nicht aufeinander abgestimmt, so dass es mitunter zu enttäuschenden Ergebnissen kommt.

Wünschenswert ist es daher ein kosmetische Kombinationsprodukt anzubieten, dass diese Probleme lösen hilft.

Die Erfindung besteht aus einem kosmetisches Kombinationsprodukt umfassend mindestens zwei Kombinationsproduktbestandteile gewählt aus
a.) ein oder mehreren Nahrungsergänzungsmitteln,
b.) ein oder mehreren kosmetischen Zubereitungen, die topisch auf der menschlichen Haut applizierbar sind, und/oder
c.) ein oder mehreren selbstklebenden Hautauflagen.
Das Kombinationsprodukt bzw. deren Bestandteile, das Ergänzungsmittel, die Zubereitungen und/oder die Hautauflagen beinhalten dabei ein oder mehrere gleiche Wirkstoffe. D.h. die Ergänzungsmittel a.), die Zubereitungen b.) und ggf. die Hautauflagen c.) enthalten jeweils einen oder mehrere Wirkstoffe, von denen mindestens einer auch in den anderen Kombinationsproduktbestandteilen a.), b.) und/oder c.) enthalten ist.

Weiterhin kann das Kombinationsprodukt bzw. deren Bestandteile, das Ergänzungsmittel, die Zubereitungen und/oder die Hautauflagen zur kosmetischen Behandlung, Pflege oder Verschönerung des gleichen Typus angewendet werden, z.B. alle dienen gegen Faltenbildung oder alle dienen als Anti-Cellulitemittel.
Weiterhin kann das Kombinationsprodukt bzw. deren Bestandteile, das Ergänzungsmittel, die Zubereitungen und/oder die Hautauflagen räumlich zueinander angeordnet sein, wie z.B. nebeneinander in einem Verkaufsregal oder durch eine Kombinationspackung, die zwei oder mehrere der Bestandteile umfasst. Der Anwender erhält somit ein aufeinander abgestimmtes Kosmetikkonzept ohne in verschiedenen Läden, Abteilungen, Regalen die zueinander passsenden Mittel zu suchen.
Ebenso ist es erfindungsgemäß das Kombinationsprodukt bzw. deren Bestandteile so aufeinander abzustimmen, dass sie zeitlich aufeinander abgestimmt, z.B. in bestimmter Reihenfolge, angewendet werden können.

Bevorzugt umfasst das Kombinationsprodukt ein Nahrungsergänzungsmittel und eine kosmetische Zubereitung.
In einer weiteren besonders bevorzugten Ausführungsform umfasst das Kombinationsprodukt ein Nahrungsergänzungsmittel, eine kosmetische Zubereitung und eine selbstklebende Hautauflage.

So kann der Anwender beispielsweise morgens ein erfindungsgemäßes Nahrungsergänzungsmittel in Form einer Kapsel einnehmen (schlucken), über den Tag die zu behandelnden Hautpartien mit dem Kombinationsproduktbestandteil der Zubereitung eincremen und abends, z.B. zum Schlafen, die entsprechende Hautauflage auflegen.
Er hat somit ein abgestimmtes Konzept zur Hand, das ihm allumfassende Hautpflege gewährleistet. Zudem werden Unverträglichkeiten vermieden, da alle Kombinationsproduktbestandteile den oder die gleichen Wirkstoffe beinhalten.

Der Anwender erzielt gerade durch diese Kombination eine mehr als zufrieden stellende Wirkung, weil er praktisch tagein tagaus das Problem bekämpft. Auch lassen sich die Produkte nach individuellen Vorlieben anwenden, z.B. 2x täglich eine Kapsel und Verzicht auf die selbstklebende Hautauflage oder mehrmals eincremen und Hautauflage während der Arbeit und zum Schlafen gehen.
Das erfindungsgemäße Kombinationsprodukt ist für diese individuelle Anwendungsweise dahingehend vorteilhaft, da die Bestandteile den oder die gleichen Wirkstoffe beinhalten und aufeinander abgestimmt sind (zeitlich, räumlich), so dass mögliche Nebenwirkungen, Unverträglichkeiten, Fehlanwendungen etc. auf ein Minimum verringert werden
So auch z.B. aufgrund der verschärften Sicherheitsvorkehrungen bei Reisen. Sollte der Anwender die kosmetische Zubereitung aufgrund der strengen Handgepäckkontrolle im Flugsicherheitsbereich entsorgen müssen, so bleiben ihm immer noch die einnehmbaren Kapseln und das selbstklebende Hautauflage. Die Behandlung wird also nicht unterbrochen.

Der Vorteil eines Patches liegt beispielweise in der über acht Stunden kontinuierlichen Wirkstoffabgabe der Pflaster. Außerdem können diese auch nachts angewendet werden

Bevorzugte kosmetische Wirkstoffe für das oder die Nahrungsergänzungsmittel können gewählt werden aus der Gruppe Carnitin und/oder dessen Derivate, insbesondere L-Carnitin-tartrat, Tee-Extrakte (Grüner Tee), Mate-Tee ( Ilex Paraguariensis), weißer Tee-Extrakte (Camelia Sinensis), Kräuterteeextrakt, Ubichinon Q10, Kreatin, Kreatinin, Taurin, Koffein, Flavonoide, Vitamin E , Mate-Teeextrakt, konjugierte Linolsäure (CLA), Safflower Extrakt, Koffein, Panthotensäure, B-Vitamine, Biotin, Aminosäure und/oder deren Derivate und/oder Folsäure.

Als kosmetisch wirksam werden erfindunsgemäß Stoffe angesehen, die topisch appliziert und/oder systemisch zu einer Hautpflege und/oder-verschönerung beitragen.

Besonders bevorzugt ist Carnitin oder deren Derivate, insbesondere L-Carnitin-tartrat, als Wirkstoff in zwei oder drei der Kombinationsproduktbestandteilen auszuwählen.

Carnitin, 3-Hydroxy-4-(trimethylammonium)-buttersäurebetain, der Struktur

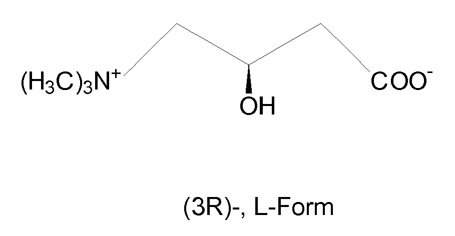

Die L-Form des Carnitins ist in tierischen Geweben weit verbreitet und ein charakteristischer Bestandteil der gestreiften Muskulatur v. a in dunklen Fleischsorten. In pflanzlichen Lebensmitteln wie Obst, Gemüse und Getreide ist L-Carnitin nur in geringen Mengen (<4 mg/100 g) enhalten.

Der Gesamtbestand an L-Carnitin im menschlichen Körper beträgt etwa 20-25 g. In Herz- und Skelettmuskulatur sind 98% der Reserven gespeichert.

L-Carnitin fungiert als Carrier-Molekül beim Transport langkettiger Fettsäuren durch die innere Mitochondrien-Membran in den mitochondrialen Matrixraum, während mittel- und kurzkettige Fettsäuren diese auch ohne eine Veresterung mit L-Carnitins passiern können.

L-Carnitin wird in zahlreichen Produkten auch zur Nahrungsergänzung angeboten. Zielgruppen sind (Ausdauer-)Sportler sowie übergewichtige Personen, denen L-Carnitin zur Leistungsteigerung bzw. als Schlankheitsmittel ("Fat-Burner") angeboten wird. Die Wirksamkeit ist in beiden Fällen sehr umstritten. Da bei gesunden Menschen ein L-Carnitin-Mangel sehr selten ist, ist von einer Carnitin-Supplementierung kein Nutzen zu erwarten. Carnitin wird bei seiner biochemischen Funktion als Carrier nicht verbraucht, so dass eine Umsatzsteigerung im Bereich des Fettstoffwechsels nicht zu einem Carnitin-Mehrbedarf führt. Umgekehrt führt eine zusätzliche Carnitin-Aufnahme nicht zu einer Steigerung der Fettsäure-Oxidation. Ein Zuviel an Carnitin wird über die Niere wieder ausgeschieden.

Bei kardiovakulären Krankheiten kann L-Carnitin durch einen Anstieg der β-Oxidation der Fettsäuren, erhöhte ATP-Level, eine Reduzierung der Blut- und Gewebefettwerte (freie Fettsäuren) sowie durch eine Steigerung der Durchblutung des Herzens die Herzleistung verbessern und insgesamt die Belastbarkeit des Herzens erhöhen. Zudem wird L-Carnitin eine gewisse immunstimmulierende Funktion zugeschrieben, die auf eine Erhöhung der Aktivität der Granulocyten, T-Lymphocyten und Killerzellen zurückgeführt wird.

Überraschenderweise hat sich gezeigt, dass eine selbstklebende Matrix enthaltend Carnitin sowie eine topisch applizierbare kosmetische Zubereitung enthaltend L-Carnitin sich positiv auf die Reduktion der Cellulite auswirkt. Die Lymphzirkulation wird angeregt.

Das erfindungsgemäße Kombinationsprodukt enthaltend bevorzugt Carnitin ist daher zur Pflege der durch Cellulite beeinflussten Hautpartien geeignet.

Die Verwendung des Kombinationsprodukts zur Behandlung der durch Cellulite verursachten Hauterscheinungen ist damit bevorzugt.

Der Wirkstoff L-Carnitin-tartrat ist bevorzugt in mindestens zwei der Kombinationsproduktbestandteilen, bevorzugt in dreien, Nahrungsergänzungsmittel, Zubereitung und Hautauflage, enthalten.

Safflower Extrakt (Distelöl) ist ein Extrakt oder Öl mit hohem Gehalt an Linolsäure. Diese mehrfach ungesättigte Fettsäure bewahrt die Haut vor der Austrocknung.

Die konjugierten Linolsäuren (engl. *conjugated linoleic acids* oder abgekürzt CLA, CAS-Nummer: 2420-56-6, Formel: C18H32O2) sind eine Fettsäure-Gruppe mit acht geometrischen Isomeren, von denen bestimmte Isomere besonders in Fleisch- und Milchprodukten von Wiederkäuern vorkommen.

Es gibt mehrere verschiedene CLA-Isomere: Das 9,11-Isomer (cis9, trans11 - Zwischenprodukt der biologischen Hydrierung mehrfach ungesättigter Fettsäuren in Pansen von Wiederkäuern) trägt besonders zum Wachstum von Muskeln bei. Das 10,12-Isomer verhindert Lipogenese (Speichern von Fett in der Fettzelle). Erhältliche CLA Produkte enthalten normalerweise beide Isomere zu gleichen Anteilen.

Studien an Menschen belegen, dass CLA den Körperfettanteil reduziert, während gleichzeitig der Muskelanteil erhöht wird.

Besonders bevorzugt ist konjugierte Linolsäure, gewonnen aus dem Öl der Färberdistel oder anderen pflanzlichen Ölen oder deren Derivate, insbesondere als Triglycerid.

Der Mate-Strauch *(Ilex paraguariensis)* wächst wild als Unterholz in den Araukarien-Wäldern Brasiliens, Uruguays und Paraguays, auf dem Hochland zwischen den Flüssen Paranä, Paraguay, Uruguay und ihren Zubringern, also ist seine Heimat der Urwald des Paranäbeckens. Noch heute wird jedoch etwa die Hälfte des auf dem Markt befindlichen Mate von wildwachsenden Bäumen geerntet.

*Mate* ist ein in Südamerika weit verbreitetes Aufgussgetränk, das durch Aufguss kleingeschnittener trockener Blätter des *Ilex paraguayensis* gewonnen wird. Mate bezeichnet ursprünglich das Trinkgefäß (aus dem Quechua *mati* für Trinkgefäß), heutzutage auch das Getränk, das von den Guarani *caiguá* genannt wurde. Die Blätter hingegen werden als *yerba* (span.: Kraut) bezeichnet.

Heutzutage wird Mate oft mit "Gaucho-Tradition" in Verbindung gebracht, ist aber tatsächlich schon vor der Kolonisierung von den Ureinwohnern Südamerikas getrunken worden.

Ursprünglich und heute wieder in den nördlicheren, wärmeren Gebieten des südlichen Südamerika wird der Sud als Tereré kalt getrunken (im Sommer sehr erfrischend).

Teilweise werden in Südamerika auch andere Aufgussgetränke als Mate bezeichnet, z.B. *mate de coca,* bereitet aus den Blättern des Cocastrauchs.

In den frischen Blättern sind 0,35 bis 1,7 % Koffein, etwa 0,1 bis 0,2 % Theobromin, Theophyllin und 4 bis 16 % Gerbstoffe enthalten. Der Aufguss ist gelbgrün und enthält abhängig von der Stärke Koffein, Theobromin, Chlorophyll, Gerbsäure, ätherisches Öl und Vanillin. An Vitaminen finden sich die Vitamine A, B1, B2 und C. Das Aroma grünen und gerösteten Mates wurde gaschromatographisch getrennt und per Massenspektrometrie analysiert, dabei wurden von mehr als 250 Komponenten etwa 200 identifiziert. In absteigender Reihenfolge wurden Ketone, Aldehyde und aliphatische Alkohole, heterocyclische Verbindungen, Carbonsäuren und Lactone, Terpenalkohole, Furanone, Phenole, aromatische Verbindungen, Pyrazine und Pyrrole nachgewiesen. Pyrrole, Pyrazine und Furanone entstehen vor allem bei der Maillard-Reaktion während des Röstvorganges.

Panthotensäure gehört zu den Vitaminen des B-Komplexes.

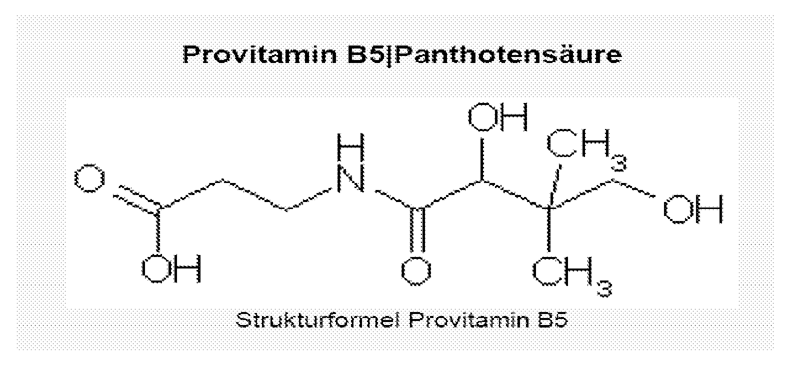

Sie ist leicht wasserlöslich und hitzeempfindlich. In Nahrungsmitteln kommt Pantothensäure meist in gebundener Form als Bestandteil des Coenzym A vor. Sie kann nicht im Körper gespeichert, muss also täglich mit der Nahrung aufgenommen werden. Pantothensäure ist hauptsächlich an enzymatischen Reaktionen in Stoffwechselvorgängen beteiligt. Im Zellstoffwechsel dient sie der Energiegewinnung. Darüber hinaus trägt zum Aufbau von verschiedenen Neurotransmittern, Fettsäuren, Kohlehydraten, Cholesterin, Hämoglobin und der Vitamine A und D bei.

Die Zufuhrempfehlung zur Vermeidung von Mangelerscheinungen liegt derzeit bei 6 mg täglich. Pantothensäure ist in vielen pflanzlichen und tierischen Nahrungsmitteln vorhanden. Leber, Vollkornprodukte und Eiern haben einen hohen Gehalt.

Da nahezu jedes Nahrungsmittel Pantothensäure enthält, ist ein Mangel sehr selten. Darmerkrankungen wie beispielsweise Colitis ulcerosa und Alkoholabhängigkeit können allerdings zu einem Mangel führen. Auch für Dialysepatienten und Diabetiker besteht die Möglichkeit eines Mangels an diesem Vitamin.

Neben oder anstelle des bevorzugten Carnitin und/oder CLA können weitere geeignete Wirkstoffe im Sinne der Erfindung in den Kombinationsproduktbestandteilen entweder einzeln oder auch in Kombination beigefügt werden, insbesondere Wirkstoffe, die den Zustand der Haut positiv beeinflussen. So zeigte sich, dass Wirkstoffe zur positiven Beeinflussung der Altershaut, die die Entstehung von Falten oder auch bestehenden Falten vermindern. Als besonders bevorzugte Wirkstoffe gelten daher Biochinone, insbesondere Ubichinon Q10, Isoflavon und Isoflavonoide, Genistein, Arctiin, Cardiolipin, Liponsäure, Anti Freezing Proteine, Hopfen- und Hopfen-Malz-Extrakte, und/oder die Restrukturierung des Bindegewebes fördernde Stoffe, Isoflavonoide sowie Isoflavonoid-haltige Pflanzenextrakte wie z.B. Soja- und Klee-Extrakte, die auch in den erfindungsgemäßen Zubereitungen und Hautauflagenmatrices sehr gut verwendet werden können. Auch zeigte sich, dass sich das erfindungsgemäße Kombinationsprodukt in besonderer Weise eignet, Wirkstoffe zur Unterstützung der Hautfunktionen bei trockener Haut, wie beispielsweise Vitamin C, Biotin, Kreatin, Kreatinin, Propionsäure, Glycerin, Grüntee-Extrakte, white tea - Extrakte oder - Lösungen, zu verwenden.
In ähnlicher Weise erwies sich die Einarbeitung von Wirkstoffen zur Linderung bzw. positiven Beeinflussung von irritativen Hautzuständen, sei es bei empfindlicher Haut im allgemeinen oder bei durch Noxen gereizter Haut (UV-Licht, Chemikalien), als vorteilhaft. Hier sind Wirkstoffe zu nennen wie Sericoside, verschiedene Extrakte des Süssholzes, Licochalcone A, Silymarin bzw. Silyphos, Flavonoide, Dexpanthenol, Ethanol, Inhibitoren des Prostaglandinstoffwechsels, insbesondere der Cyclooxygenase, und des Leukotrienstoffwechsels, insbesondere der 5-Lipoxygenase, aber auch des 5-Lipoxygenase Inhibitor Proteins, FLAP. Auch erwies sich die Einarbeitung von Modulatoren der Pigmentierung als vorteilhaft. Hier sind Wirkstoffe zu nennen, die die Pigmentierung der Haut vermindern und so zu einer kosmetisch gewünschten Aufhellung der Haut führen und/oder das Auftreten von Altersflecken reduzieren und/oder bestehende Altersflecken aufhellen, wie Tyrosinsulfat, Vitamin C, Liponsäure und Liponamid, verschiedene Extrakte des Süssholzes, Kojisäure, Hydrochinon, Arbutin, Fruchtsäuren, insbesondere Alpha-Hydroxy-Säuren (AHAs), Bearberry (Uvae ursi), Ursolsäure, Ascorbinsäure, Grüntee-Extrakte, Aminoguanidin und/oder Pyridoxamin. In gleicher Weise erwiesen sich die erfindungsgemäßen Nahrungsergänzungsmittel, Zubereitungen oder Matrices als hervorragende Grundlage für Wirkstoffe, die eine verstärkte/schnellere Bräunung der Haut herbeiführen (Advanced Glycation Endproducts (AGE), Lipofuscine, Nukleinsäure-Oligonukleotide, Purine und Pyrimidine, NO-freisetzende Substanzen, sei es mit oder ohne Einfluss von UV-Licht.

Bevorzugt ist der Einsatz von Mate-oder Grüntee-Extrakt, da in Kombination mit Carnitin eine die Haut pflegende und vor allem ein die Cellulite abbauender Effekt beobachtet werden konnte.

Das Geheimnis des weißen Tees ist durch seine schonende Verarbeitung begründet, so dass dieser nahezu unverändert bleibt. White tea Extrakte enthalten einen hohen Gehalt an Polyphenolen, sie zählen zu den hochwirksamen Antioxidanzien, die freie Radikale unschädlich machen. Die Forschung, die sich mit diesen Aspekten beim weißen Tee beschäftigt, ist noch relativ jung. Im Handel sind Weiße Tees unter den Namen Yin Zhen (Silbernadel) und Yin Long (Silber Drache) erhältlich.

Bevorzugt ist daher auch der Einsatz von White Tea-Extrakt, da in Kombination mit Carnitin eine die Haut pflegende und vor allem ein die Cellulite abbauender Effekt beobachtet werden konnte.

Bevorzugt ist auch der Einsatz von Mate-Tee, Coenzym Q10, wärmenden Stoffen und Panthenol. Das Kombinationsprodukt kann auf jeden Fall auch zur Erreichung eines ebenmäßigen Hautbildes eingesetzt werden.

Nahrungsergänzungsmittel sind Konzentrate von Nährstoffen oder sonstigen Stoffen mit ernährungsspezifischer oder physiologischer Wirkung, die in dosierter Form in den Verkehr gebracht werden und dazu bestimmt sind, die allgemeine Ernährung zu ergänzen. Erfindungsgemäß werden insbesondere Nahrungsergänzungsmittel bzw. die darin enthaltenen Wirkstoffe so gewählt, dass eine kosmetische Wirkung, d.h. Pflege oder Verschönerung der Haut, zu beobachten ist.

Neben den zuvor genannten Wirkstoffen können weiterhin die folgenden bevorzugten Wirkstoffe gewählt werden aus der Gruppe Carotionoide, Catechine, Isoflavonoide aus Soja oder Rotklee, Calcium, Zink, Selen, Eisen, Magnesium, Silicium, Kieselerde, Algen, Guarana, Cranberry, Cacaoextrakte, Kollagen, Hyaluronsäure, Vitamin C, Vitamin A, Fettsäuren, wie Omega-3-Fettsäuren, Omega-6-Fettsäuren, Gamma-linolensäure, ungesättigte Pflanzenöle, Taurin, Balaststoffe, Rosenwurz, Spargelextrakt, Zimtextrakte, Ingwer, Proteine, Flavonoide, Saponine, Kräuterextrakte, Gemüseextrakte und/oder Fruchtextrakte.

Neben der erfindungsgemäßen Auswahl mindestens eines Wirkstoffes, der in zwei oder mehreren Bestandteilen des Kombinationsproduktes enthalten ist, kann der Fachmann die zuvor vorteilhaft erwähnten Wirkstoffe auswählen. Dabei wählt er natürlich die Wirkstoffe auch entsprechend Ihrer Verträglichkeit aus, so dass beispielsweise in Nahrungsergänzungsmitteln andere Stoffe zusätzlich enthalten sein können als in kosmetischen Zubereitungen oder patches.

Als Wirkstoffe in den Kombinationsproduktbestandteilen werden bevorzugt ein, zwei oder bevorzugt alle Stoffe gewählt werden aus der Gruppe konjugierte Linolsäure (CLA) oder deren Derivate, L- Carnitin oder deren Derivate, insbesondere L-Carnitin-tartrat, Vitamin E und/oder Mate-Teeextrakt eingesetzt.

Als sogenannte Hilfsstoffe können in den Nahrungsergänzungsmitteln folgende Stoffe enthalten sein, die der Fachmann je nach Verwendungsform, Wirkweise und Anwendungsverfahren frei wählen kann.

Als Füllstoffe/Träger sind bevorzugt Stärkederivate (Mais-, Weizen-, Kartoffelstärke), Cellulosederivate (Hydroxypropylcellulose, Methylcellulose, Fructose, Glucose, Mannitol, Sorbitol, Ethylcellulose, Natriumcarboxymethylcellulose), Lactose, Pflanzenöle, Sojaöl, Neutralöl, pflanzliche und synthetische Mono-di-und Triglyceride, Wachse und/oder Lipide zu wählen.
Als Bindemittel sind bevorzugt Trockenbindemittel, Polyethylenglykole, Polyvinylpyrrolidon, Calciumhydrogenphosphat, PVP (Kollidon) und/oder Gelatine zu wählen.

Als Gleitmittel/Fließregulierungsmittel/Trennmittel sind bevorzugt Magnesiumstearat, Calciumbehenat, Glycerinmonostearat, Pflanzenfette, hochdisperses Siliciumdioxid (Aerosil), Lecithin, Calciumcarbonat, Calciumphosphat, Magnesiumphosphat, Mannit, Cellulose, Salze der Speisefettsäuren, Magnesiumsalze der Speisefettsäuren, Thermoxidiertes Sojaöl verestert mit Mono- und Diglyceriden, Kaliumcarbonate, Magnesiumcarbonate, Magnesiumhydroxid" Magnesiumoxid, Natriumferrocyanid, Kaliumferrocyanid, Calciumferrocyanid, Calciumsilikat, Magnesiumsilikat, Talkum, Aluminiumsilikate, Fettsäuren, Bienenwachs, Candelillawachs, Carnaubawachs und/oder Mikrokristallines Wachs zu wählen.

Als Farbstoffe eignen sich bevorzugt:
gelb: Curcumin; Riboflavin; Phosphatester; Tartrazin; Chinolingelb
orange: Gelborange S
rot: Karmin; Azorubin; Amaranth; Cochenillerot A; Erythrosin; Rot 2G; Allurarot AC
blau: Patentblau V; Indigotin; Brillantblau FCF
grün: Chlorophyll; Kupferhaltige Komplexe der Chlorophylle und Chlorophylline; Grün S
gelb, orange und rot: Carotine; Annatto; Capsanthin; Lycopin; Beta-apo-8'-Carotinal; E 160f - Beta-apo-8'-Carotinalethylester; Lutein; Canthaxanthin; Zeaxanthin; Betanin; Anthocyane weiß, rot, Metalle: Calciumcarbonat; E 171 - Titandioxid; E 172 - Eisenoxid; E 173 - Aluminium; E 174-Silber; E 175-Gold

Als Emulgatoren sind bevorzugt Lecithin, Propylenglycolalginat, Polysorbat 20, Polysorbat 80, Polysorbat 40, Polysorbat 60, Polysorbat 65, Ammoniumphosphatide, Hydroypropylcellulose, Hydroxypropylmethylcellulose, Salze der Speisefettsäuren, Mono- und Diglyceride von Speisefettsäuren, Essigsäureester von Mono- und Diglyceriden von Fettsäuren, Milchsäureester von Mono- und Diglyceriden von Fettsäuren, Citronensäureester von Mono- und Diglyceriden von Fettsäuren, Weinsäureester von Mono- und Diglyceriden von Fettsäuren, Diacetylweinsäureester von Mono- und Diglyceriden von Fettsäuren, Essig-Weinsäureester von Mono- und Diglyceriden von Fettsäuren, Zuckerester von Speisefettsäuren, Zuckerglyceride, Polyglycerinester von Speisefettsäuren, Polyglycerin-Polyricinoleat, Propylenglycolester von Speisefettsäuren, Thermoxidiertes Sojaöl verestert mit Mono- und Diglyceriden, Natriumstearoyl-2-lactylatCalciumstearoyl-2-lactylat, Stearyltartrat, Sorbitanmonostearat, Sorbitantristearat, Sorbitanmonolaurat, Sorbitanmonooleat, Sorbitanmonopalmitat, Hydroxypropylstärke, Hydroxypropyldistärkephosphat und/oder Stärkenatriumoctenylsuccinat zu wählen.

Als Geliermittel, Verdickungsmittel und Feuchthaltemittel sind bevorzugt Alginsäure, Alginate, Agar Agar, Carrageen, Stärke und Stärkederivate (z.B. Monostärkephosphat, Phosphatiertes Distärkephosphat, Acetyliertes Distärkephosphat, Acetylierte Stärke, Acetyliertes Distärkeadipat, Hydroxypropylstärke, Hydroxypropyldistärkephosphat, Stärkenatriumoctenylsuccinat) Johannisbrotkernmehl, Guarkernmehl, Traganth, Gummi arabicum; Xanthan; Karayagummi, Tarakernmehl, Gellan, Sorbit, Mannit, Glycerin, Konjakwurzel, Pektin, Cellulose und Cellulosederivate ( z.B. Methylcellulose, Hydroypropylcellulose, Hydroxypropylmethyl-cellulose, Methylethylcellulose, NatriumCarboxymethylcellulose, vernetzte Carboxymethylcellulose), Salze der Speisefettsäuren, Magnesiumsalze der Speisefettsäuren, Essigsäureester von Mono- und Diglyceriden von Fettsäuren, Fettsäuren, Bienenwachs, Candelillawachs, Carnaubawachs, Schellack, Mikrokristallines Wachs und/oder Hydriertes Poly-1-decen zu wählen.

Als Süßungsmittel/Aromen sind bevorzugt Sorbit, Mannit, Aspartam, Cyclamat, Isomalt, Saccharin, Sucralose, Thaumatin, Neohesperidin DC, Maltit, Lactit, Xylit, Fructose, Mannitol, Saccharose, Lactose, Glucose, Sorbitol, künstliche und synthetische Aromen und/oder Aromazubereitungen zu wählen.

Das erfindungsgemäße Nahrungsergänzungsmittel liegt bevorzugt in Form einer Kapsel vor, insbesondere in Form einer Weichgelatinekapsel vor.

Weitere Darreichungsformen sind bevorzugt Hartgelatinekapseln, Pulver, Granulate, Kügelchen, Pastillen, Tabletten, Brausetabletten, Lutschtabletten, überzogene Tabletten (Dragees), Buccaltabletten, Kautabletten, Subligualtabletten, Bonbons, Kaugummis, Lösungen, Tinkturen, Emulsionen, Säfte, Konzentrate, Sirup und/oder Trinkampullen.
Die entsprechenden Verpackungen oder Versieglungen, Blister, Tuben etc., sind aus dem Stand der Technik bekannt.
Das Nahrungsergänzungsmittel liegt bevorzugt in einer portionierten Form zur Einmal-Einnahme vor, wie z.B. als Kapsel, Dragee oder als Pulver. Die Kapseln oder Dragees sind beispielsweise in einer Blisterverpackung verschweißt. In Pulverform kann das Ergänzungsmittel beispielweise in Papiertütchen verpackt werden.
Auch andere Formen, flüssig, als Bonbon (harter Hülle, weicher Kern), als Tablette etc. sind denkbar und erfindungsgemäß. Die Art und Form sind aus dem Stand der Technik bekannt.

Das Nahrungsergänzungsmittel wird bevorzugt in Blistern, Tiegeln oder Spendersysteme konfektioniert. Eine Verpackungseinheit umfasst bevorzugt eine oder 2 Monatsmengen (30 -60 Kapseln).

Als bevorzugte Wirkstoffe sind neben den zuvor genannten Wirkstoffen insbesondere konjugierter Linolsäure, Carnitin und/oder Mate-Teeextrakt enthalten. Besonders bevorzugt sind alle drei Stoffe im Nahrungsergänzungsmittel enthalten. Vorteilhaft dabei in Kombination mit Vitamin E und Lecithin.
Erfindungsgemäß umfassen in diesem Fall die kosmetische Zubereitung dann vorteilhaft z.B. nur Carnitintartrat, Carnitintartrat und andere kosmetische Wirkstoffe, wie z.B. Folsäure oder Q10, Carnitintartrat und Vitamin E oder aber auch alle drei Carnitintartrat, Mate-Teeextrakt und Vitamin E.
Erfindungsgemäß bevorzugt ist natürlich, wenn alle drei Bestandteile, das Nahrungsergänzungsmittel, die kosmetische Zubereitung und die Hautauflage, beispielsweise Carnitintartrat und Vitamin E umfassen.

Das bevorzugt kapselförmige Nahrungsergänzungmittel umfasst ein oder mehrere Wirkstoffe, von denen zumindest ein, bevorzugt alle, Wirkstoffe auch in den anderen Kombinationsproduktbestandteilen der Zubereitung und/oder der Hautauflage enthalten sind. Darüber hinaus umfasst das Nahrungsergänzungsmittel als Grundstoffe bevorzugt Sojaöl, Lecithin, Glycerin, Gelantine, Titandioxid und Eisenoxid.

Beispielsweise umfassen ein oder mehrere Nahrungsergänzungsmittel die folgenden Ingredientien, wie sie in der Tabelle 1 dargestellt sind.

**Tabelle 1. bevorzugt Ingredientien des Nahrungsergänzungsmittels a.)**

| **Funktion** | **Bestandteil** | **Handelsbezeichnung** | **Hersteller/Lieferant** |
|---|---|---|---|
| Wirkstoff | Conjugated Linoleic Acid 200-250mg | Tonalin® TG 80 | Cognis |
| Wirkstoff | L-Carnitin | Carnipure® tartrate micronized | Lonza |
| Wirkstoff | Mate fol extr.s.siccum 30-50mg | EFLA® 920 | Frutarom Switzerland Ltd. |
| Wirkstoff | Vitamin E 5-15 I.U. | z.B.D,L-Alpha-Tocopherol USP (E 307) | z.B. Cognis, DSM |
| Hilfsstoff | Lecithin (E 322) 0.5-200mg | Sternlecithin B-10 Special | Solae Europe, S.A |

Die Tabelle 1 offenbart zudem die bevorzugten Mengen der Ingredientien und von wem sie bezogen werden können.

**Tabelle 2:**

| Beispielrezeptur 1 - Füllmasse ohne Gelatinehülle | |
|---|---|
| Wirkstoffe Nahrungsergänzungsmittel | w (mg) |
| Conjugated Linoleic Acid | 200 |
| Vitamin E | 5 |
| L-Carnitine (Tartrat) | 40 |
| Green mate leaf powder extract | 40 |
| Lecithin from Soy bean, E 322 | 5,000 |

| Beispielrezeptur 2 - Nahrungsergänzungsmittel mit Weichgelatinehülle | |
|---|---|
| Inhaltstoffe Nahrungsergänzungsmittel | w (mg) |
| Conjugated Linoleic Acid, Triglyceride | 300,000 |
| RRR-alpha-Tocopherol Concentrate | 20,000 |
| L-Carnitine-Tatrate | 80,000 |
| Green mate leaf powder extract | 100,000 |
| Soya-Bean Oil, hydrogenated, Ph.Eur. | 30,795 |
| Lecithin from Soy bean, E 322 | 7,000 |
| Glycerol 85%, Ph.Eur. | 60,00 |
| Gelatin, 200 Bloom, Ph.Eur.,NF | 115,00 |
| Titanium Dioxide, Ph.Eur.,USP, E 171, C.I.-No.77891 | 2,04 |
| Red Iron Oxide, E 172, C.I.-No.77491, NF | 1,02 |

| Beispielrezeptur 3 - Nahrungsergänzungsmittel mit Weichgelatinehülle | |
|---|---|
| Inhaltstoffe Nahrungsergänzungsmittel | w (mg) |
| Conjugated Linoleic Acid, Triglyceride | 200,000 |
| RRR-alpha-Tocopherol Concentrate | 5,225 |
| L-Carnitine-Tatrate | 43,980 |
| Green mate leaf powder extract | 40,000 |
| Soya-Bean Oil, hydrogenated, Ph.Eur. | 30,795 |
| Lecithin from Soy bean, E 322 | 5,000 |
| Glycerol 85%, Ph.Eur. | 58,73 |
| Gelatin, 200 Bloom, Ph.Eur.,NF | 114,90 |
| Titanium Dioxide, Ph.Eur.,USP, E 171, C.I.-No.77891 | 2,04 |
| Red Iron Oxide, E 172, C.I.-No.77491, NF | 0,51 |

| Beispielrezeptur 4 Nahrungsergänzungsmittel mit Weichgelatinehülle | |
|---|---|
| Inhaltstoffe Nahrungsergänzungsmittel | w (mg) |
| Conjugated Linoleic Acid, Triglyceride | 100,00 |
| RRR-alpha-Tocopherol Concentrate | 2,220 |
| L-Carnitine-Tatrate | 20,000 |
| Green mate leaf powder extract | 1,000 |
| Soya-Bean Oil, hydrogenated, Ph.Eur. | 30,795 |
| Lecithin from Soy bean, E 322 | 2,000 |
| Glycerol 85%, Ph.Eur. | 58,73 |
| Gelatin, 200 Bloom, Ph.Eur.,NF | 114,90 |
| Titanium Dioxide, Ph.Eur.,USP, E 171, C.I.-No.77891 | 4,04 |
| Red Iron Oxide, E 172, C.I.-No.77491, NF | 0,20 |

Die bevorzugten Menge pro Nahrungsergänzungsmittel sind
- CLA (Conjugated Linoleic acid), Tagesdosis 10 mg-2 g, bevorzugt 50 mg-1 g, besonders bevorzugt 200-600mg
- Vitamin E, Tagesdosis: 2 mg-800 mg, bevorzugt 2.5 mg-200 g, besonders bevorzugt 5-20 mg
- L-Carnitin, Tagesdosis: 5 mg-3 g, bevorzugt 10 mg-200 mg, besonders bevorzugt 10-100 mg
- Lecithin, entsprechend einem Cholingehalt < 500mg

Die bevorzugte Einnahmeempfehlung liegt bei 1-10 Kapseln, besonders bevorzugt ist die tägliche Einnahme von 1-3 Kapseln.

Die erfindungsgemäße kosmetische Zubereitung kann bevorzugt entsprechend der Offenbarung der DE 102005032237 aufgebaut sein. Der Inhalt der DE 102005032237 ist hiermit explizit als Offenbarung der vorliegenden Erfindung anzusehen.

Bevorzugt umfasst die erfindungsgemäße Zubereitung als Kombinationsproduktbestandteil ein oder mehrere Wirkstoffe, von denen dann zumindest ein, bevorzugt alle, Wirkstoffe auch in den anderen Kombinationsproduktbestandteilen des oder der Nahrungsergänzungsmittel und/oder der Hautauflage(n) enthalten sind.

Die Wirkstoffgehalt der kosmetischen Zubereitung liegt vorteilhaft in einer Gesamtkonzentration von 0,0001 bis 12,5 Gewichts-% und bevorzugt in einer Gesamtkonzentration von 0,01 bis 0,8 Gewichts-%, jeweils bezogen auf das Gesamtgewicht der Zubereitung.

Darüber hinaus umfasst die kosmetische Zubereitung als Grundstoffe vorteilhaft Polyacrylsäuresalze, Acrylat/C10-30 Alkylacrylat Crosspolymere und/oder Carrageenan.

Das erfindungsgemäß bevorzugte Polyacrylsäuresalz ist dabei das Natriumpolyacrylat (CAS: 9003-04-7) welches beispielsweise von der Firma Cognis unter dem Handelsnamen COSMEDIA SP angeboten wird. Diese Polyacrylsäuresalze sind nicht vernetzt.

Es ist erfindungsgemäß vorteilhaft, als quervernetzte Polyacrylsäuresalze Carbomere einzusetzen. Das erfindungsgemäß bevorzugte quervernetzte Polyacrylsäuresalz ist dabei das Natriumcarbomer (z.B: CAS: 9007-16-3, CAS:9007-17-4, CAS:9062-04-8, CAS:76050-42-5) welches beispielsweise von der Firma Noveon unter dem Handelsnamen Carbopol 980 angeboten wird.

Ein erfindungsgemäß bevorzugtes Acrylat/C10-30 Alkylacrylat Crosspolymer (INCI: Acrylates/C10-30 Alkylacrylates Crosspolymer) wird beispielsweise von der Firma Noveon unter dem Handelsnamen Pemulen TR-1 angeboten.

Das erfindungsgemäß bevorzugte Carrageenan ist unter der CAS-Nr.: 9000-07-1 (EINECS-Nr.: 232-524-2) abgelegt und kann beispielsweise unter dem Handelsnamen Gelcarin GP-379NF bei der Firma Interorgana erworben werden.

Erfindungsgemäß vorteilhafte Ausführungsformen der erfindungsgemäßen kosmetischen Zubereitung sind dadurch gekennzeichnet, dass sie als weitere Komponente 0,01 bis 2,5 Gewichts-% Vinylpyrrolidon/Vinylacetat-Copolymer (VP/VA-Copolymer), bezogen auf das Gesamtgewicht der kosmetischen Zubereitung enthält.

Das erfindungsgemäß bevorzugte Vinylpyrrolidon/Vinylacetat-Copolymer (VP/VA-Copolymer) ist ein Polymer, welches zu 60% aus Vinylpyrrolidon und zu 40% aus Vinylacetat gebildet wird und beispielsweise unter dem Handelsnamen Luviskol VA64 W von der Firma BASF angeboten wird.

Die erfindungsgemäße Zubereitung ist erfindungsgemäß vorteilhaft dadurch gekennzeichnet, dass das Gewichtsverhältnis von Polyacrylsäuresalzen zu Acrylates/C10-30 Alkylacrylates Crosspolymer von 1 :10 bis 10 : 1 und bevorzugt von 1 :1 bis 2 :1 beträgt.

Die erfindungsgemäße Zubereitung ist erfindungsgemäß vorteilhaft dadurch gekennzeichnet, dass das Gewichtsverhältnis von Polyacrylsäuresalzen zu Carrageenan von 1 :10 bis 10 :1 beträgt.

Die erfindungsgemäße Zubereitung ist erfindungsgemäß vorteilhaft dadurch gekennzeichnet, dass das Gewichtsverhältnis von Polyacrylsäuresalzen zu Vinylpyrrolidon/Vinylacetat-Copolymeren von 1:10 bis 10 :1 und bevorzugt von 1:1 bis 2 :1 beträgt.

Erfindungsgemäß ist auch eine kosmetische Zubereitung, welche die erfindungsgemäße Emulgatorkombination aus Polyacrylsäuresalzen, quervernetzten Polyacrylsäuresalzen, Acrylat/C10-30 Alkylacrylat Crosspolymer und Carageenan in einer Gesamtkonzentration von 0,05 bis 9,5 Gewichts-% und bevorzugt in einer Konzentration von 0,5 bis 1,8 Gewichts-%, jeweils bezogen auf das Gesamtgewicht der kosmetischen Zubereitung, enthält.

Dabei handelt es sich erfindungsgemäß bevorzugt bei der kosmetischen Zubereitung um ein wässriges oder wässrig-alkoholisches Gel oder eine Gelcreme, erfindungsgemäß vereinfacht als Gel bezeichnet.

Gelcremes sind besonders leichte Produkte mit einem niedrigen Emulgator- und Lipidgehalt. Sie zeichnen sich dadurch aus, dass sie sich leicht auf der Haut verteilen lassen und ein Frischegefühl vermitteln. Nach dem Produktauftrag soll auf der Haut kein oder nur wenig Rückstand verbleiben. Gelcremes enthalten in der Regel einen relativ hohen Anteil an hydrophilen Verdickungsmitteln. Da sich der Verdicker oder das Verdickersystem in der äußeren Phase befindet, hat es einen signifikanten Einfluß auf die sensorischen Eigenschaften des Produktes. Gängige Verdickersysteme lassen sich entweder nicht leicht verteilen, ergeben kein Frischegefühl oder hinterlassen einen zu klebrigen Rückstand auf der Haut.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder - monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte und/oder Wirkstoffe wie Selbstbräuner, Repellentien, Vitamine, Pflanzenextrakte, Niacinamid, Panthenol.

Erfindungsgemäß bevorzugt enthält die erfindungsgemäße Zubereitung als weitere Bestandteile Alpha-Hydroxysäuren und/oder deren Salze. Dabei werden erfindungsgemäß bevorzugt Milchsäure/Lactate oder Zitronensäure/Citrate in einer Konzentration von 0,01 bis 5 Gewichts-%, bezogen auf das Gesamtgewicht der Zubereitung eingesetzt.

Erfindungsgemäß vorteilhaft kann die erfindungsgemäße Zubereitung weitere kosmetische Zusatzstoffe enthalten, beispielsweise die nach der Kosmetikverordnung zugelassenen UV-Lichtschutzfilter, und Konservierungsmittel bzw. Konservierungshelfer. Derartige Wirkstoffe können erfindungsgemäß vorteilhaft in Konzentrationen von 0,01 bis 30 Gew-%, bezogen auf das Gesamtgewicht der Zubereitung in dieser enthalten sein.
Die erfindungsgemäße kosmetischen Zubereitung enthält erfindungsgemäß vorteilhaft mindestens einen wasserlöslichen Farbstoff.

Die erfindungsgemäße kosmetischen Zubereitung weist erfindungsgemäß vorteilhaft ein transparentes oder transluzentes Aussehen auf. Sie kann erfindungsgemäß vorteilhaft in einer transparenten Verpackung aufbewahrt werden.

Es ist vorteilhaft im Sinne der vorliegenden Erfindung, wenn die erfindungsgemäße kosmetische Zubereitung frei ist von Tensiden mit einem HLB-Wert von größer 25. Es ist erfindungsgemäß bevorzugt, wenn die erfindungsgemäße kosmetische Zubereitung frei ist von Tensiden mit einem HLB-Wert von größer 20. Die HLB-Werte von Tensiden können den üblichen Tabellenwerken (z.B. Fiedler, Lexikon der Hilfsstoffe für Pharmazie, Kosmetik und angrenzende Gebiete, Editio Cantor Verlag, Aulendorf) entnommen werden.

Erfindungsgemäß ist auch eine Emulsion aus einer kosmetischen Zubereitung (bevorzugt aus einem wässrigen oder wässrig-alkoholischem Gel) und hauteigenen Lipiden. Eine derartige Emulsion wird dabei dadurch hergestellt, dass die kosmetische Zubereitung (bevorzugt aus einem wässrigen und wässrig-alkoholischem Gel oder Gelcreme) auf die Haut aufgetragen und anschließend verrieben wird.

Das Verfahren zur Herstellung einer derartigen erfindungsgemäßen Emulsion, welches dadurch gekennzeichnet ist, dass eine erfindungsgemäße kosmetische Zubereitung (bevorzugt aus einem wässrigen oder wässrig-alkoholischem Gel oder Gelcreme) auf die Haut aufgetragen und anschließend verrieben wird, ist erfindungsgemäß. Dabei wird das Verreiben der kosmetischen Zubereitung auf der Haut bevorzugt mit den Händen oder Fingern durchgeführt, kann jedoch auch mit Hilfe eines Applikators (beispielsweise eines Schwämmchens oder eines Massagekopfes) erfindungsgemäß erfolgen.

Die Zubereitung kann erfindungsgemäß vorteilhaft in Form einer dünnflüssigen, sprühfähigen wässrigen oder wässrig-alkoholischen Lösung, in Form eines Gels, als Salbe, Creme oder Lotion (ggf. sprühbar) vorliegen. Bevorzugt liegt die Zubereitung als Gel vor.

Die Zubereitung kann erfindungsgemäß vorteilhaft auch als Spray oder als Tränkungsmedium für ein Pflaster oder Tuch eingesetzt werden. Daher sind auch Pflaster und Tücher getränkt mit der erfindungsgemäßen Zubereitung, erfindungsgemäß wobei hier ein Übergang zum Kombinationsproduktbestandteil der Hautauflage gegeben ist, sofern eine Selbstklebung vorgesehen ist.

Die erfindungsgemäße selbstklebende Hautauflage umfasst eine auf der menschlichen Haut haftende Matrix und mindestens einen kosmetischen Wirkstoff, wobei der Wirkstoff bevorzugt in der Matrix enthaltend ist.
Der oder die bzw. einer oder mehrere der in der Matrix der Hautauflage enthaltenen Wirkstoffe sind dann erfindunsgemäß auch in den anderen Kombinationsproduktbestandteilen des Nahrungsergänzungsmittels und/oder der Zubereitung enthalten.

Bevorzugt ist die erfindungsgemäße Hautauflage entsprechend den in der DE 102005053909 beschriebenen Auflagen ausgestaltet. Die Offenbarung der DE 102005053909 ist hiermit Bestandteil der vorliegenden Erfindung.

Unter der erfindungsgemäßen Hautauflage werden alle kosmetisch anwendbaren Auflagen wie Patch, Pad, Tücher, Pflaster, Dressings, Cataplasm, Bandagen, Masken verstanden.
Erfindungsgemäß ist hierbei bevorzugt nicht ein medizinisch wirksamer Wirkstoff sondern ein kosmetisch wirksamer Stoff in der Haftmatrix enthalten.

Als selbstklebende Matrix werden in Wasser gelbildende Polymere, Polyisobutylene oder Cataplasmen bevorzugt. Bevorzugt ist insbesondere eine Klebemasse basierend auf Polyacrylsäure bzw. Polyacrylaten.
Der Anteil an in Wasser gelbildendem Polymer wie z.B. Polyacrylsäuregel in der Matrix regelt das Haftvermögen. Insbesondere die in DE 10260873 und DE 10056010 offenbarten Matrizes sind hiermit Bestandteil der vorliegenden Erfindung.

Erfindungsgemäß vorteilhafte Polyacrylate sind Acrylat-Alkylacrylat-Copolymere, insbesondere solche, die aus der Gruppe der so genannten Carbomere oder Carbopole (Carbopol® ist eine eingetragene Marke der B. F. Goodrich Company) gewählt werden. Insbesondere zeichnen sich das oder die erfindungsgemäß vorteilhaften Acrylat-Alkylacrylat-Copolymere durch die folgende Struktur aus:

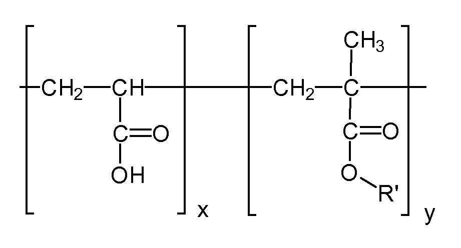

Darin stellen R' einen Alkylrest, insbesondere einen langkettigen Rest, und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkylacrylat-Copolymere, welche unter den Handelbezeichnungen Carbopol® 1382, Carbopol® 981 und Carbopol® 5984 von der B. F.Goodrich Company erhältlich sind, bevorzugt Polyacrylate aus der Gruppe der Carbopole der Typen 980, 981, 1382, 2984, 5984 sowie besonders bevorzugt Carbomer 2001.
Ferner vorteilhaft sind Copolymere aus C₁₀₋₃₀-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Das in Wasser gelbildende Polymer, insbesondere Polyacrylsäure und/oder deren Copolymere, werden bevorzugt in einer Menge von 2 - 55 Gew.%, besonders bevorzugt zwischen 5-30 Gew.% eingesetzt.

Die Herstellung der Polymermatrices erfolgt bevorzugt ohne Verwendung organischer Lösemittel, vorzugsweise bei 40 - 95°C, in handelsüblichen Mischern/Knetern oder kontinuierlich in geeigneten Extrudern.
Als in Wasser gelbildendes Polymer eignet sich u.a. auch Affenbrotbaummehl.
Vorteilhaft hat sich beispielsweise die Kombination von in Wasser gelbildendem Polymer (Polyacrylsäure), Meeresalgenextrakt, wie Alginate und/oder Agar-Agar, und ein- oder mehrwertigem Alkohol gezeigt. Auf diese Weise lassen sich unter Verwendung von Wasser, in Wasser gelbildendem Polymer, Meeresalgenextrakt und ein- oder mehrwertigem Alkohol als Ausgangsmaterialien gezielt weiche, geschmeidige, selbstklebende Hydrogelmatrices als Basis zur Herstellung und Anwendung als Pflaster, TTS, Cataplasmen oder kosmetischen Pads / Matrices herstellen.
Zur Ausfertigung besonderer anwendungstechnischer Eigenschaften können die Polymermatrices mit entsprechenden Weichmachern, Lösungsvermittlern, Penetrationsenhancern, Neutralisationsmitteln wie z.B. Tromethamol (2-Amino-2-(hydroxymethyl)-1,3-propandiol), Triethanolamin (2,2',2"-Nitrilotriethanol) oder NaOH, Füllstoffen und/oder anderen bekannten Zusätzen versetzt werden, deren Zusatz jedoch nicht zwingend ist.
In einer erfindungsgemäß besonders bevorzugten Ausführungsform enthält die Polymermatrix bzw. Gelmatrix dermatologische oder kosmetische Wirkstoffe zur kontrollierten lokalen bzw. systemischen Abgabe an/in die Haut, in Mengen von insgesamt bis zu 35 Gew.%, bevorzugt bis zu 15 Gew.%, insbesondere bis zu 2 Gew.%.
Da es sich bei der erfindungsgemäßen Matrix ggf. auch um eine wasserhaltige Applikationsform handelt, erreicht man zusätzlich einen kühlenden Effekt, der *per se* schon kosmetisch angenehm ist und zum Wohlbefinden beiträgt. Diese positive Wirkung kann durch die Zugabe weiterer pflegender Bestandteile verstärkt werden. Neben Glycerin können insbesondere Serinol (3-Amino-1,2-Propandiol) bzw. Isoserinol (2-Amino-1,3-Propandiol) sowie Harnstoff und PCA (Pyrrolidoncarbonsäure) als feuchtigkeitsspendende Substanzen beigefügt werden. Selbstverständlich können auch weitere Substanzen zu diesem Zwecke beigefügt werden.

Als erfindungsgemäßes Matrixsystem wird auch Polyisobutylene PIB bevorzugt eingesetzt.

Als weitere Matrices sind neben PIB Polyisobutylen hydrophobe Basispolymere wie SIS (Styrol/Isopren/Styrol)-Triblockcopolymere, SBS (Styrol/Butadien/Styrol)-Triblockcopolymere, SBR (Copolymere aus Styrol und Butadien), synthetische und/oder natürliche Polyisoprene, Polyamid, Polyester, Co-Polyester, Polyurethane und/oder Mischungen daraus möglich. Aus der Vielzahl bekannter Polymermatrizes sind Polyacrylate und Polyisobutylene besonders bevorzugt.
Polyisobutylene erfüllen als Matrixgrundlage die Anforderungen einer selbstklebenden, hautschonenden und schmerzfrei ablösbaren Polymermatrix besonders gut, so dass es folgerichtig ist, die Polyisobutylene bevorzugt als Matrixgrundlage auszuwählen.

SBR ist eine Sammelbezeichnung für Copolymere aus Styrol und Butadien, die die beiden Monomere meistens im Gewichtsverhältnis von ca. 23,5:76,5, in Ausnahmefällen auch von 40:60 enthalten und deren Makromoleküle überwiegend die Struktureinheiten I und II aufweisen:

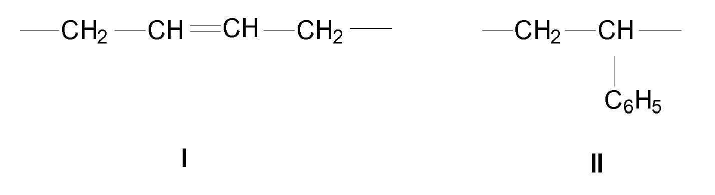

Erfindungsgemäße wasserhaltige Matrices können dazu benutzt werden um sehr trockene Hautareale mit Feuchtigkeit zu versorgen.
Damit ist die erfindungsgemäße Polymermatrix als Pflaster, Pad oder Hautauflage zur Pflege der Haut und insbesondere zu einfachen Kühlungszwecken außerordentlich gut geeignet und, zudem selbstklebend ausgerüstet, einfach anzuwenden.

Vorteilhaft ist auch, entsprechend zur Vermeidung der Nachteile aus dem Stand der Technik, dass die Polymermatrix lösemittelfrei ist.

Das erfindungsgemäße Kombinationsprodukt, umfassend bevorzugt mindestens ein Nahrungsergänzungsmittel und mindestens eine kosmetische Zubereitung sowie gegebenfalls eine selbstklebende Hautauflage ist zur kosmetischen Prophylaxe oder kosmetischen Behandlung von Hautunebenheiten, und/oder Cellulite besonders predestiniert.
Die Verwendung der Kombinationsproduktbestandteile zur Herstellung eines Kombinationspräparates zur Pflege und/oder Verschönerung der menschlichen Haut ist ebenfalls bevorzugt.

Unter Prophylaxe und Behandlung wird im Rahmen dieser Offenbarung ausschließlich die kosmetische Prophylaxe und Behandlung verstanden und keinesfalls eine therapeutische Prophylaxe und Behandlung im Sinne des Patentrechts.

Die erfindungsgemäße kosmetische Prophylaxe oder kosmetische Behandlung von Cellulite kann erfindungsgemäß vorteilhaft in Kombination mit Massagegeräten, Ultraschall, Infrarotlicht, Textilien, Bandagen und/oder okklusiven Folien erfolgen.

Der Vorteil und die damit bevorzugte Verwendung des Kombinationsproduktes liegt darin, dass alle Bestandteile zur gleichen kosmetischen Behandlung, Pflege und/oder Verschönerung der menschlichen Haut angewendet werden können. D.h. die alle Bestandteile werden zur Behandlung der durch Cellulite verursachten Hauterscheinungen angewendet.

### Beispiele

Kombinationsproduktbestandteil - kosmetische Zubereitung in Form von Gelen

| | **1** | **2** | **3** | **4** | **5** |
|---|---|---|---|---|---|
| Polyacrylsäure, Na-Salz (Carbomer) | 1,0 | 0,2 | 0,5 | 0,1 | 1,5 |
| PEG-40-Stearat | 0,5 | --- | 0,75 | -- | -- |
| Carrageenan | 1,25 | 0,2 | 0,2 | 0,1 | 2,5 |
| Natriumpolyacrylat | 0,2 | 0,2 | 0,3 | 0,5 | 1,5 |
| Vinylpyrrolidon/Vinylacetat-Copolymeren | 1,0 | --- | 0,2 | --- | --- |
| Acrylates/C10-C30 Alkyl Acrylate Crosspolymer | 1,5 | 0,2 | 0,15 | 1,5 | 0,75 |
| wasserlösliche Farbe | 0,01 | 0,4 | --- | --- | --- |
| Cyclomethicon | 1 | 8,5 | 5 | 0,5 | 3 |
| Dimethiconol | 0,5 | 0,5 | 0,75 | 1,25 | 1,0 |
| Cetylstearylalkohol | --- | --- | 1 | 1 | --- |
| hydriertes Polyisobuten | 0,5 | --- | 1,0 | --- | 0,25 |
| Carnitin | 0,5 | 0,1 | 0,75 | 3,0 | 0,25 |
| Panthenol | 0,5 | --- | 0,75 | 0,25 | 0,1 |
| Glycerin | 3,0 | 8,6 | 12,5 | 17,2 | 5,0 |
| Parfum | q,s, | q,s, | q,s, | q,s, | q,s, |
| Methylpropandiol | 4,5 | 2,0 | 0,5 | 1,5 | --- |
| Propylparaben | 0,3 | --- | 0,25 | 0,15 | --- |
| Ethanol | --- | 10,0 | 5,0 | --- | 3,5 |
| Wasser | ad 100 | ad 100 | ad 100 | ad 100 | ad 100 |

Mit dem in Tabelle 2 beispielhaft aufgeführten Nahrungsergänzungsmittel ergibt sich in Kombination mit einer kosmetischen Zubereitung nach Beispiel 3 eine vorteilhaftes Kombinationsprodukt, das zur Bekämpfung der sichtbaren Hautverändeurngen durch Cellulite angewendet werden kann.

So kann das Gel nach dem Duschen täglich auf die betroffenen Hautpartien aufgetragen werden und das Nahrungsergänzungsmittel nach der Mahlzeit, z.B. Frühstück, eingenommen werden.
Eine zeitlich aufeinander abgestimmte Abfolge der Anwendung der Kombinationsproduktbestandteile ist erfindungsgemäß vorzugeben. So kann der Anwender beispielsweise morgens nach dem Aufstehen die betroffenen Hautpartien mit der erfindungsgemäßen Gelzubereitung eincremen, nach dem Frühstück die Kapsel schlucken und zur Nacht eine entsprechende Hautauflage applizieren.

Die Hautauflage ist besonders bevorzugt in einer flächiger Ausführungsform mit einer Gesamtfläche von 0,2 bis 1000 cm² ausgestattet. Bevorzugt in einer Fläche von 8 bis 15 cm zu 10 bis 20 cm. Damit werden beispielsweise großflächige Bereiche (bis zu 1000 cm²) zur Behandlung der Orangenhaut an den Oberschenkeln abgedeckt.
Bevorzugt ist dabei die Hautauflage mit einem Polymermatrixgewichtsanteil von 0,1 bis 1000 g, insbesondere von 14 g pro Hautauflage ausgestattet. Die Form der Auflage kann dabei rund, oval, eckig oder den Hautpartien angepasst gestaltet sein.

## Patentansprüche

1. Kosmetisches Kombinationsprodukt umfassend mindestens zwei Bestandteile gewählt aus
a.) ein oder mehreren Nahrungsergänzungsmitteln,
b.) ein oder mehreren topisch applizierbaren kosmetischen Zubereitungen und/oder
c.) ein oder mehreren selbstklebenden Hautauflagen,
**dadurch gekennzeichnet, dass**
die Ergänzungsmittel a.), die Zubereitungen b.) und/oder die Hautauflagen c.) jeweils einen oder mehrere kosmetisch wirksame Wirkstoffe enthalten, von denen mindestens einer auch in den anderen Bestandteilen a.), b.) und/oder c.) enthalten ist.

2. Kombinationsprodukt nach Anspruch 1, **dadurch gekennzeichnet, dass** mindestens zwei der Bestandteile a.), b.) und/oder c.) räumlich benachbart angeordnet sind.

3. Kombinationsprodukt nach Anspruch 1 oder 2 umfassend mindestens ein Nahrungsergänzungsmittel und mindestens eine kosmetische Zubereitung als Gel oder Emulsion.

4. Kombinationsprodukt nach Anspruch 1, 2 oder 3 umfassend mindestens ein Nahrungsergänzungsmittel, mindestens eine kosmetische Zubereitung und mindestens eine selbstklebende Hautauflage.

5. Kombinationsprodukt nach einem der vorstehenden Ansprüche, **dadurch gekennzeichnet, dass** als Wirkstoff ein oder mehrer Stoffe gewählt werden aus der Gruppe Carnitin und/oder deren Derivate, insbesondere L-Carnitin-tartrat, Tee-Extrakte (Grüner Tee), Mate-Tee ( Ilex Paraguariensis), weißer Tee (Camelia Sinensis), Kräuterteeextrakt, Ubichinon Q10, Kreatin, Kreatinin, Taurin, Koffein, Flavonoide, Vitamin E, Mate-Teeextrakt, konjugierte Linolsäure (CLA), Safflower Extrakt, Koffein, Panthotensäure, B-Vitamine, Biotin, Aminosäure und/oder deren Derivate und/oder Folsäure.

6. Kombinationsprodukt nach Anspruch 5, **dadurch gekennzeichnet, dass** als Wirkstoff ein, zwei oder bevorzugt alle Stoffe gewählt werden aus der Gruppe konjugierte Linolsäure (CLA) oder deren Derivate, L- Carnitin oder deren Derivate, insbesondere L-Carnitin-tartrat, Vitamin E und/oder Mate-Teeextrakt.

7. Kombinationsprodukt nach einem der vorstehenden Ansprüche, **dadurch gekennzeichnet, dass** als Wirkstoff L-Carnitin in mindestens zwei der Kombinationsproduktbestandteile enthalten ist.

8. Kombinationsprodukt nach einem der vorstehenden Ansprüche **dadurch gekennzeichnet, dass** das Nahrungsergänzungsmittel neben ein oder mehreren Wirkstoffen Sojaöl, Lecithin, Glycerin, Gelantine, Titandioxid und/oder Eisenoxid umfasst.

9. Kombinationsprodukt nach einem der vorstehenden Ansprüche mit einem Nahrungsergänzungsmittel als Weichgelatinekapseln, Hartgelatinekapseln, Pulver, Granulate, Kügelchen, Pastillen, Tabletten, Brausetabletten, Lutschtabletten, überzogene Tabletten (Dragees), Buccaltabletten, Kautabletten, Subligualtabletten, Bonbons, Kaugummis, Lösungen, Tinkturen, Emulsionen, Saft, Konzentrate, Sirup und/oder Trinkampulle.

10. Kombinationsprodukt nach einem der vorstehenden Ansprüche **dadurch gekennzeichnet, dass** das Nahrungsergänzungsmittel in Form von Weichgelatinekapseln vorliegt.

11. Kombinationsprodukt nach einem der vorstehenden Ansprüche **dadurch gekennzeichnet, dass** das Nahrungsergänzungsmittel in Form in Blistern, Tiegeln oder Spendersysteme konfektioniert wird.

12. Kombinationsprodukt nach einem der vorstehenden Ansprüche **dadurch gekennzeichnet, dass** die kosmetische Zubereitung neben ein oder mehreren Wirkstoffen Polyacrylsäuresalze, Acrylat/C10-30 Alkylacrylat Crosspolymere und/oder Carrageenan umfasst.

13. Kombinationsprodukt nach einem der vorstehenden Ansprüche **dadurch gekennzeichnet, dass** der oder die Wirkstoffe der Hautauflage in der Klebmatrix der Auflage enthaltend sind.

14. Kombinationsprodukt nach einem der vorstehenden Ansprüche **dadurch gekennzeichnet, dass** die selbstklebende Hautauflage neben ein oder mehreren Wirkstoffen als Matrix Polyacrylate oder Polyisobutylene umfasst.

15. Verwendung des Kombinationsproduktes nach einem der vorstehenden Ansprüche, **dadurch gekennzeichnet, dass** alle Bestandteile zur gleichen kosmetischen Behandlung, Pflege und/oder Verschönerung der menschlichen Haut angewendet werden können.

16. Verwendung des Kombinationsproduktes nach einem der vorstehenden Ansprüche, **dadurch gekennzeichnet, dass** die Anwendung der Bestandteile a.), b.) und/oder c.) zeitlich aufeinander abgestimmt sind.

17. Verwendung des Kombinationsproduktes nach einem der vorstehenden Ansprüche zur kosmetischen Prophylaxe oder kosmetischen Behandlung von Hautunebenheiten und/oder Cellulite.

18. Verwendung mindestens zweier Bestandteile gewählt aus
a.) ein oder mehreren Nahrungsergänzungsmitteln,
b.) ein oder mehreren topisch applizierbaren kosmetischen Zubereitungen und/oder
c.) ein oder mehreren selbstklebenden Hautauflagen,
wobei die Ergänzungsmittel a.), die Zubereitungen b.) und/oder die Hautauflagen c.) jeweils einen oder mehrere Wirkstoffe enthalten, von denen mindestens einer auch in den anderen Bestandteilen a.), b.) und/oder c.) enthalten ist, zur Herstellung eines Kombinationspräparates zur Pflege und/oder Verschönerung der menschlichen Haut.
